# EUROPÄISCHE PATENTSCHRIFT

(11) **EP 1 238 646 B1**
(45) Veröffentlichungstag und Bekanntmachung des Hinweises auf die Patenterteilung: **21.06.2006**
(21) Anmeldenummer: 02004613.2
(22) Anmeldetag: 28.02.2002
(51) Int. Cl.: A61K 8/81, A61K 8/86, A61Q 5/06

(54) **Haarbehandlungsmittel**
Hair care product
Produit de traitement capillaire

(30) Priorität: 01.03.2001 DE 10109820
(43) Veröffentlichungstag der Anmeldung: 11.09.2002
(73) Patentinhaber: KPSS-Kao Professional Salon Services GmbH, 64297 Darmstadt (DE)
(72) Erfinder: Link, Thorsten, Dr., 64319 Pfungstadt (DE); Weichaus, Dirk, 64404 Bickenbach (DE); Cajan, Christine, 56130 Bad Ems (DE)

(56) Entgegenhaltungen:
- EP-A- 0 897 711
- EP-A- 0 985 405
- US-A- 5 972 356
- US-A1- 2001 002 254

## Beschreibung

Die vorliegende Erfindung betrifft ein Haarbehandlungsmittel in Form eines wäßrigen Gels mit verbesserten rheologischen Eigenschaften.

Es ist seit Jahrzehnten bekannt, Haarbehandlungsmittel wie Festigungs- und Konditioniermittel der verschiedensten Art zu benutzen. Sie enthalten in der Regel filmbildende Wirkstoffe wie natürliche und synthetische Polymere und werden insbesondere als Lösungen, Emulsionen, Gele oder Lotionen eingesetzt, die auch als Aerosole oder Pumpsprays zum Einsatz gelangen können.

Entsprechende Zusammensetzungen, die in der Regel nach der Applikation im Haar verbleiben, sind beispielsweise in der Monographie von K. Schrader, Grundlagen und Rezepturen der Kosmetika, 2. Aufl. (Hüthig Buch Verlag, 1989), auf den Seiten 737 bis 760 beschrieben.

Besonders bevorzugt ist hierbei die Verwendung von gelförmigen Produkten. Hier stellt sich jedoch das Problem, daß die filmbildenden Substanzen für sich allein keine ausreichende verdickende Wirkung ausüben; andererseits die zusätzliche Mitverwendung von Verdickungsmitteln zu Inkompatibilitäten führen kann.

Die vorliegende Erfindung geht daher von der Aufgabenstellung aus, viskositätsstabile gelförmige Haarbehandlungsmittel zur Verfügung zu stellen, die außerdem eine befriedigende haarkonditionierende bzw. haarfestigende Wirkung ausüben, gut in das Haar einmassiert werden können und dem Haar auch Glanz und Volumen verleihen.

Diese Aufgabe wird durch ein Haarbehandlungsmittel gelöst, daß in Form eines wasserlöslichen Gels vorliegt, und eine Kombination aus
a) 1 bis 10 Gew.-% mindestens eines neutralisierten, filmbildenden Copolymerisats aus Methylmethacrylat n-Butyl- oder tert.-Butylacrylat und (Meth)Acrylsäure und
b) 0,25 bis 5 Gew.-% mindestens eines neutralisierten Copolymerisats aus mindestens einem Monomeren der allgemeinen Formel (I)

   **CH**_{**2**}**=CR**^{**1**}**R**^{**2**} **(I),**

   wobei R¹ für eine Gruppe A-(CH₂CH₂O)ₓ-R³ steht, 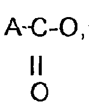 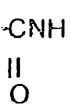 und CH₂O bedeutet, x eine Zahl von 1 bis 50, vorzugsweise von 5 bis 30 ist, R³ einen C₈-C₂₄₋Alkylrest, vorzugsweise einen C₁₂- bis C₁₈-Alkylrest, R² H, CH₃ und CH₂COOH bedeuten, und einer α,β-ungesättigten Carbonsäure sowie gegebenenfalls weiteren polymerisierbaren Verbindungen enthält,
wobei der pH-Wert des Mittels 6 bis 8 berträgt.

Der Viskositätsbereich des erfindungsgemäßen Mittels liegt bei 50 000 bis 150 000 mPa.s, gemessen bei 20°C (Brookfield RVT; Heliopath Spindel C 10UpM).

Dieses Produkt verleiht dem Haar nicht nur Glanz, Fülle und Volumen, einen vollen Griff, Spannkraft und verbesserte Naß- und Trockenkämmbarkeit, sondern weist auch keinerlei sonst bei solchen Produkten häufig auftretende Klebrigkeit oder Hygroskopizität auf.
Darüber hinaus ist dieses Gel äußerst homogen und bis 50°C viskositätsstabil; es kann auch bei Normaltemperatur durch einfaches Zusammenrühren der Bestandteile hergestellt werden.

Der bevorzugte Anteil an den Copolymeren a) liegt bei etwa 2 bis etwa 7,5, insbesondere etwa 2,5 bis etwa 6 Gew.-%, berechnet auf die Gesamtzusammensetzung.
Dabei beträgt der Anteil der Carbonsäure vorzugsweise etwa 5 bis 20 % des Copolymeren.

Derartige Copolymere werden durch Copolymerisation der entsprechenden Monomeren gewonnen und sind auch auf dem Markt erhältlich, z.B. unter den Handelsnamen "Balance 0/55 ®", "Balance CR®" und "Balance Extra®" der Fa. National Starch Chemical Co., "Luvimer®" der BASF AG oder "Carbose®" der Fa. Goodrich. Sie sind im CTFA International Cosmetic Ingredient Dictionary unter der generischen Bezeichnung "Acrylates Copolymers", und speziell "Acrylic/Acrylate Copolymer" gelistet. In den erfindungsgemäßen Zusammensetzungen liegen sie beispielsweise als Alkali-, Ammonium- oder Alkanolaminsalze von.

Der Bestandteil b) der erfindungsgemäßen Zusammensetzung ist vorzugsweise in einer Menge von 0,5 bis 4, insbesondere 1 bis 2,5 Gew.-%, berechnet auf die Gesamtzusammensetzung, enthalten.

Bevorzugte Bestandteile b) sind Copolymerisate aus Acrylsäure, Methacrylsäure und Itaconsäure mit Estern dieser Säuren mit ethoxylierten C₈-C₂₄-, insbesondere C₁₂-C₂₂-Fettalkoholen, wobei die bevorzugte Zahl x der Ethylenoxideinheiten in der Formel (I) zwischen 5 bis 30 liegt.

Geeignete Copolymere sind beispielsweise Acryl- oder Methacrylsäure/ Acryl- oder Methacrylsäurepolyethoxyalkylester-Copolymere des Typs Acrylates/ Steareth-20 Methacrylate Copolymer, wie sie von der Firma Rohm and Haas Co. unter der Bezeichnung "Acrysol-22®" und "Aculyn-22®", oder Acryl- oder Methacrylsäure/Polyethoxyalkylallylether-Copolymere des Typs Steareth-10 Allyl Ether/Acrylates-Copolymer, wie sie von der Firma Allied Colloids unter der Bezeichnung "Salcare SC90®" oder Acryl- oder Methacrylsäure/ ltaconsäurepolyethoxyalkyfester Copolymere des Typs Acrylates/ Steareth-20 Itaconate Copolymer und Acrylates/ Ceteth-20 Itaconate Copolymer, wie sie von der Firma National Starch Co. unter der Bezeichnung "Structure 2001®" und "Structure 300®" vertrieben werden.

Weitere in diesem Zusammenhang geeignete Copolymere sind solche aus (Meth)Acrylsäure und einem Methacrylsäureester eines mit 25 Ethylenoxid-Einheiten ethoxylierten Behenylalkohols, bekannt unter dem Handelsnamen "Aculyn 28®, und die in der EP 0870 785 A1 beschriebenen Produkte, beispielsweise ein unter dem Namen "Synthaler®" erhältliches Acrylates/ Palmeth-25 Acrylate Copolymer.

Die erfindungsgemäßen gelförmigen Zusammensetzungen können die in solchen Mitteln üblichen Stoffe enthalten, soweit diese die Stabilität des Gels nicht beeinträchtigen.
Solche Stoffe sind z.B. flüssige Fette und Öle mit einem Schmelzpunkt von maximal etwa 30°C, vorzugsweise etwa 25°C, natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, Purcellinöl (Cetearyloctanoat) oder auch Oliven- bzw. Sojaöl, sowie sonstige Mono-, Di- und Triglyceride, Lanolin und dessen Derivate, ebenso Mineralöle wie Paraffinöl und Vaseline, synthetische Öle und Wachse wie Silikonöle, Polyethylenglykole, sowie weitere hydrophobe Komponenten, z.B. Fettsäureester wie Ethyl-, n-Propyl- und Isopropylmyristat, -palmitat, -stearat, -laurat, -oleat und -isostearat, Decyloleat, Oleyloleat, Isocetylstearat, Hexyllaurat, Decylstearat, Dibutyladipat, Oleyladipat, Dioctyladipat, Tristearylcitrat, Myristylmyristat, Cetylpropionat, Oleylerucat, Polyethylenglykol- und Polyglycerylfettsäureester wie PEG-7-glycerylcocoat, etc. Weitere geeignete Substanzen sind Fettalkohole wie Oleylalkohol, Decyl- und Laurylalkohol und natürliche Gemische derselben wie Cocosfettalkohol, die auch ethoxyliert sein können.
Falls erwünscht, können oberflächenaktive Stoffe, insbesondere nichtionische, amphotere oder zwitterionische, zugesetzt werden. Solche nichtionischen Tenside sind beispielsweise Fettalkoholpolyglykolether.
Besonders geeignete C₁₀-C₂₂-Fettalkoholether sind die unter den Trivialnamen "Laureth" "Myristeth" "Oleth" , "Ceteth", "Deceth", "Steareth", "Trideceth" und "Ceteareth" nach der CTFA-Nomenklatur mit Anfügung der Zahl der Ethylenoxid-Moleküle bezeichneten Alkylpolyglykolether, z.B. "Laureth-16" und "Trideceth-6".
Der durchschnittliche Ethoxylierungsgrad liegt dabei zwischen etwa 2,5 und etwa 25, vorzugsweise etwa 5 und etwa 20.

Geeignete weitere nichtionische Tenside sind Verbindungen aus der Klasse der Alkylpolyglucoside mit der allgemeinen Formel

R-O-(CH₂CH₂O)ₙ-Zₓ,

worin R eine Alkylgruppe mit 8 bis 20, vorzugsweise 10 bis 14 Kohlenstoffatomen, Zₓ einen Saccharidrest mit 5 bis 6 Kohlenstoffatomen, n, eine Zahl von 0 bis 10, und x eine Zahl zwischen 1 und 5, vorzugsweise 1,1 bis 2,5 bedeuten.
Andere zusätzlich mitverwendbare nichtionische Tenside sind z.B. die verschiedenen Sorbitanester wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics® " im Verkehr sind.
Weitere einsetzbare nichtionische Tenside sind Aminoxide.
Solche Aminoxide gehören seit langem zum Stand der Technik, beispielsweise C₁₂₋C₁₈-Alkyldimethylaminoxide wie Lauryldimethylaminoxid, C₁₂-C₁₈-Alkylamidopropyl- oder -ethylaminoxide, C₁₂-C₁₈-Alkyldi(hydroxyethyl)- oder -(hydroxypropy)aminoxide, oder auch Aminoxide mit Ethylenoxid- und/oder Propylenoxidgruppen in der Alkylkette.
Geeignete Aminoxide sind beispielsweise unter den Bezeichnungen "Ammonyx®", "Aromox®", oder "Genaminox®" im Handel. Geeignete amphotere bzw. zwitterionische Tenside sind insbesondere die verschiedenen bekannten Betaine wie Fettsäureamidoalkylbetaine und Sulfobetaine, beispielsweise Laurylhydroxysulfobetain; auch langkettige Alkylaminosäuren wie Cocoaminoacetat, Cocoaminopropionat und Natriumcocoamphopropionat und -acetat haben sich als geeignet erwiesen.

Weitere einsetzbare Stoffe sind die verschiedenen quaternären Ammoniumverbindungen, haarkonditionierende kationische Polymere, sowie auch nichtionische, amphotere und/oder zwitterionische Polymere.
Diese Polymeren sind dem Fachmann bekannt und auf dem Markt erhältlich.

Das erfindungsgemäße Haarbehandlungsmittel kann als weiteren Bestandteil noch mindestens eine Verbindung, ausgewählt aus der Gruppe 1-Methoxypropanol(-2), 1-Ethoxypropanol(-2), Diethylenglykolmonomethyl- oder -ethylether, Dipropylenglykol-monomethyl- oder -ethylether, Benzylalkohol, Benzyloxyethanol, Phenylethylalkohol, Phenoxyethanol und/oder Zimtalkohol, vorzugsweise in einer Menge von 0,5 bis 25, insbesondere 1 bis 20, vor allem 2,5 bis 15 Gew.-%, berechnet auf die Gesamt-zusammensetzung des Mittels, enthalten.
Bevorzugte Verbindungen aus dieser Gruppe sind Ethoxydiglykol und Benzyloxyethanol.

Ein weiterer möglicher Bestandteil in diesen Mitteln ist Harnstoff, vorzugsweise in einer Menge von etwa 1 bis 10, insbesondere etwa 2,5 bis 7,5 Gew.-% des erfindungsgemäßen Mittels.

Die erfindungsgemäßen Mittel können neben den essentiellen zusätzlich auch noch weitere konditionierende wasserlösliche Eiweißhydrolysate und Polypeptide, z.B. Keratinhydrolysate, Kollagenhydrolysate vom Typ Nutrilan®" oder Elastinhydrolysate sowie insbesondere auch pflanzliche, gegebenenfalls kationisierte Eiweißhydrolysate, z.B. "Gluadin^{R"}, enthalten.

Des weiteren können alle in solchen Zusammensetzungen üblichen Bestandteile anwesend sein.
Als solche seien beispielhaft Komplexbildner, Farbstoffe, Konservierungsmittel, pH-Regler, Duftstoffe, Perlglanzmittel, Verdickungsmittel, Feuchthaltemittel, Cholesterine wie Lecithin und dessen Derivate, etc. genannt.
Eine Auflistung solcher Zusatzstoffe findet sich ebenfalls bei Schrader, I.c., auf S.695 bis 722.
Schließlich können auch noch bekannte Polysiloxane als zusätzliche konditionierende Mittel in den erfindungsgemäßen Haarbehandlungsmitteln mitverwendet werden. Deren bevorzugter Anteil liegt dabei etwa zwischen 0,5 und etwa 5, insbesondere 1 bis 3 Gew.-% der Gesamtzusammensetzung.

Geeignet sind sowohl leichtflüchtige als auch schwerffüchtige cyclische oder lineare Polysiloxane, beispielsweise die unter den Trivialnamen "Dimethicone" bzw. "Phenyldimethicone" sowie "Cyclomethicone" bekannten Silikonöle, sowie beispielsweise auch die in der EP-A 398 177 beschriebenen Silikonderivate, die dort in Kombination mit Alkylpolyglucosiden in flüssigen Detergens-Zusammensetzungen eingesetzt werden.

Ein weiterer möglicher Bestandteil in den erfindungsgemäßen Mitteln sind Pflanzenextrakte in einer Menge von etwa 0,01 bis etwa 10, vorzugsweise 0,1 bis 7,5, insbesondere 0,5 bis 5 Gew.-%, berechnet als Trockenrückstand desselben auf die Gesamtmenge des Mittels.
Ein weiterer wirkungssteigernder Bestandteil in den erfindungsgemäßen Zusammensetzungen sind Mono- und Oligosaccharide in einer Menge zwischen etwa 0,05 und 5, insbesondere 0,1 bis 4, vorzugsweise 0,25 bis 2,5 Gew.-% der Gesamtzusammensetzung.
Bevorzugte Substanzen sind dabei Glucose, Saccharose, Fructose, Maltose, Lactose, Cellobiose und insbesondere Fucose.

Im folgenden werden anhand von Ausführungsbeispielen die Erfindung und der durch sie erreichte viskositätsstabilisierende und -steigernde Effekt erläutert.

Es wurden folgende gelförmige Zusammensetzungen hergestellt

**Beispiele Nr.**

| | | 1 | 2 | 3 | 4 | 5 | 6 |
|---|---|---|---|---|---|---|---|
| DMDM-Hydantoin (Konservierungsmittel) | | 0,3 | 0,3 | 0,3 | 0,3 | 0,3 | 0,3 |
| Terpolymer aus Methylmethacrylat, n-Butylacrylat und Methacrylsäure (Balance® 0/55) | | 6,0 | 6,0 | 6,0 | 6,0 | 6,0 | - |
| Acrylates Copolymer (Luvimer® 100P) | | - | - | - | - | - | 3,0 |
| PEG-40-hydriertes Ricinusöl | | 0,3 | 0,3 | 0,3 | 0,3 | 0,3 | 0,3 |
| Parfum | | 0,3 | 0,3 | 0,3 | 0,3 | 0,3 | 0,3 |
| Aculyn® 22 | | 1,2 | - | - | - | - | 1,2 |
| Aculyn® 28 | | - | 0,7 | - | - | - | - |
| Salcare® SC 80 | | - | - | 1,5 | - | - | - |
| Synthalen® W 2000 | | - | - | - | 1,2 | | |
| Carbopor® ETD 2020 | | - | - | - | - | 1,0 | - |
| NaOH 32-%ig | adpH | 7,64 | 7,72 | 7,55 | 7,60 | 7,20 | 7,70 |
| Wasser | ad | 100,0 | 100,0 | 100,0 | 100,0 | 100,0 | 100,0 |
| Viskosität Sp. C 10 UpM (20°C) | | 155.000 | 71.000 | 72.000 | 76.200 | 15.400 | 52.700 mPa·s |

| | | | | | | | |
|---|---|---|---|---|---|---|---|
| Aculyn® 22: Acrylates/Steareth-20 Methacrylate Copolymer | | | | | | | |
| Aculyn® 28: Acrylates/Beheneth-25 Methacrylate Copolymer | | | | | | | |
| Salcare® SC 80 Steareth-10 Allyl Ether/Acrylates Copolymer | | | | | | | |
| Synthalen® W 2000: Acrylates/Palmeth-25 Acrylate Copolymer | | | | | | | |
| Carbopol® ETD 2020: Acrylates/C10-30 Alkyl Acrylate Crosspolymer | | | | | | | |
| * incht erfindungsgemäß | | | | | | | |

Diese Beispiele zeigen die überraschende Viskositätssteigerung beim Einsatz der erfindungsgemäßen Mischung aus speziellem Filmbildner a) und Verdickungsmittel b) nach den Beispielen 2 bis 4 und 6, im Vergleich mit einer bekannten Kombination nach dem Stand der Technik (Beispiel 5) mit dem Verdickungsmittel Carbopol^{R} ETD 2020, einem vernetzten (Meth) Acrylsäure/ C₁₀-C₃₀-Alkylacrylat/ Allylsucroseether-Copolymerisat.
Darüber hinaus lassen sich die die erfindungsgemäßen Zusammensetzungen gut in das Haar einmassieren; das behandelte Haar zeigte einen dezenten Glanz und eine anhaltende Formstabilität der Frisur.

## Patentansprüche

1. Gelförmiges Haarbehandlungsmittel auf wäßriger Basis, enthaltend eine Kombination aus
a) 1 bis 10 Gew.-% mindestens eines neutralisierten, filmbildenden Copolymerisats aus Methylmethacrylat n-Butyl- oder tert.-Butyl acrylat und (Meth)Acrylsäure, und
b) 0,25 bis 5 Gew.-% mindestens eines neutralisierten Copolymerisats aus mindestens einem Monomeren der allgemeinen Formel (I)
**CH**_{**2**}**=CR**^{**1**}**R**^{**2**} **(I),**
wobei R¹ für eine Gruppe A-(CH₂CH₂O)ₓ=R³ steht, 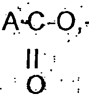 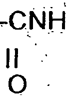 und CH₂O bedeutet, x eine Zahl von 1 bis 50, vorzugsweise von 5 bis 30 ist, R³ einen C₈-C₂₄₋Alkylrest, vorzugsweise einen C₁₂-bis C₁₈-Alkylrest, R² H, CH₃ und CH₂C0OH bedeuten, und einer α, β-ungesättigten Carbonsäure sowie gegebenenfalls weiteren polymerisierbaren Verbindungen, enthält, wobei der pH wert des Mittels 6 bis 8 beträgt, und eine Brookfield-Viskosität von 50.000 bis 150.000 mPa.s gemassen bei 20°C mit einer Helipath Spindel C und 10 UpM aufweist.

2. Mittel nach Anspruch 1 **dadurch gekennzeichnet, daß** die Komponente b) ein Copolymerisat aus (Meth)Acrylsäure und einem Allylether eines mit 5 bis 15 Mol Ethylenoxid ethoxylierten C₁₀-C₂₄-Fettalkohols darstellt.

3. Mittel nach Anspruch 1 und/oder 2, **dadurch gekennzeichnet, daß** die Komponente b) ein Copolymerisat aus (Meth)Acrylsäure und einem C₁₀-C₂₄₋Alkylpolyglykolethermethabrylat darstellt.

4. Mittel nach Anspruch 3, **dadurch gekennzeichnet, daß** Komponente b) ein Copolymerisat aus (Meth)Acrylsaure und einem C₁₂-C₁₈-Fettalkoholpolyglykolether (15-25) methacryfat darstellt.

## Claims

1. Hair treatment composition in form of a gel on aqueous basis, comprising a combination of
a) 1 % to 10 % by weight of at least one neutralized, film-forming copolymer of methyl methacrylate n-butyl or tert.-butyl acrylate and (meth)acrylic acid, and
b) 0.25 % to 5 % by weight of at least one neutralized copolymer of at least one monomer of the general formula (I)
**CH**_{**2**}**=CR**^{**1**}**R**^{**2**} **(I),**
wherein R¹ stands for a group A-(CH₂CH₂O)ₓ-R³, 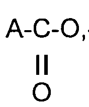 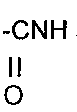 and CH₂O,
x is a number from 1 to 50, preferably 5 to 30, R³ is a C₈-C₂₄-alkyl group, preferably a C₁₂- to C₁₈-alkyl group, R² is H, CH₃ and CH₂COOH, and an α, β-unsaturated carboxylic acid as well as optionally additional compounds capable of polymerization,
whereby the composition has a pH-value of 6 to 8 and a Brookfield-viscosity of 50,000 to 150,000 mPa.s, measured at 20°C with a Heliopath spindle C 10 rpm.

2. Composition according to claim 1, **characterized in that** component b) is a copolymer of (meth)acrylic acid and an allyl ether of a C₁₀-C₂₄-fatty alcohol ethoxylated with 5 to 15 molecules of ethyleneoxide.

3. Composition according to claim 1 and/or 2, **characterized in that** component b) constitutes a copolymer of (meth)acrylic acid and a C₁₀-C₂₄-alkyl polyglycolether methacrylate.

4. Composition according to claim 3, **characterized in that** component b) is a copolymer of (meth)acrylic acid and a C₁₂-C₁₈-fatty alcohol polyglycolether (15-25) methacrylate.

## Revendications

1. Agent de traitement capillaire sous forme de gel à base aqueuse contenant une combinaison de
a) 1 à 10% en poids d'au moins un copolymère filmogène, neutralisé, de méthacrylate de méthyle, d'acrylate de n-butyle ou de tert-butyle et d'acide (méth)acrylique, et
b) 0,25 à 5% en poids d'au moins un copolymère neutralisé d'au moins un monomère de formule générale (I)
**CH**_{**2**}**=CR**^{**1**}**R**^{**2**} **(I)**
dans laquelle R¹ représente un groupe A-(CH₂CH₂O)ₓ-R³, A signifie 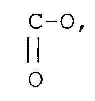 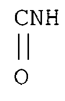 et CH₂O,
x représente un nombre de 1 à 50, valant de préférence de 5 à 30, R³ représente un radical alkyle en C₈ à C₂₄, de préférence un radical alkyle en C₁₂ à C₁₈, R² représente H, CH₃ et CH₂COOH, et d'un acide carboxylique insaturé en α, β ainsi qu'éventuellement d'autres composés polymérisables,
pour lequel la valeur de pH de l'agent vaut de 6 à 8, et il présente une viscosité Brookfield de 50 000 à 150 000 mPa.s, mesuréé à 20°C avec un appareil Heliophath, broche C, 10 tr/mn.

2. Agent selon la revendication 1, **caractérisé en ce que** le composant b) représente un copolymère d'acide (méth)acrylique et d'un éther allylique d'un alcool gras en C₁₀ à C₂₄, éthoxylé avec 5 à 15 moles d'oxyde d'éthylène.

3. Agent selon la revendication 1 et/ou 2, **caractérisé en ce que** le composant b) représente un copolymère d'acide (méth)acrylique et d'un méthacrylate d' alkylpolyglycoléther en C₁₀ à C₂₄.

4. Agent selon la revendication 3, **caractérisé en ce que** le composant b) représente un copolymère d'acide (méth)acrylique et d'un méthacrylate de polyglycoléther (15-25) d'alcool gras en C₁₂ à C₁₈.
